# EUROPEAN PATENT SPECIFICATION

(11) **EP 0 511 799 B1**
(45) Date of publication and mention of the grant of the patent: **05.06.1996**
(21) Application number: 92303752.7
(22) Date of filing: 27.04.1992
(51) Int. Cl.: C07H 17/08, A61K 31/70

(54) **Pharmaceutical compounds**
Pharmazeutische Verbindungen
Composés pharmaceutiques

(30) Priority: 29.04.1991 US 692842
(43) Date of publication of application: 04.11.1992
(73) Proprietor: ELI LILLY AND COMPANY, Indianapolis, Indiana 46285 (US)
(72) Inventor: Greene, James Michael, Indianapolis, Indiana 46217 (US); Hankins, Holly Marie, Indianapolis, Indiana 46231 (US); Stephenson, Gregory Alan, West Lafayette, Indiana 47906 (US); Wirth, David Dale, Lafayette, Indiana 47905 (US)
(74) Representative: Hudson, Christopher Mark

(56) References cited:
- EP-A- 0 208 971
- FR-A- 2 306 703
- JOURNAL OF ANTIBIOTICS. vol. XLIII, no. 10, October 1990, TOKYO JP pages 1271 - 1277; J.FIRL ET AL.: 'Epimerization of Erythromycin Derivatives'
- CHEMICAL ABSTRACTS, vol. 94, 1981, Columbus, Ohio, US; abstract no. 150536J, P.LUGER ET AL.: 'Molecular Structure of 9-deoxy 11-deoxy-9-11-(imino(2-(2-methoxyethoxy)ethylide ne)oxy)-(9S)-erythromycin, a new Erythromycin Derivative' page 87 ;column 1 ;

## Description

This invention concerns semi-synthetic pharmaceutical chemistry, and relates to novel solvate forms of a macrolide antibiotic. More particularly, this invention provides the acetone, 1-propanol, 2-propanol, and 1-butanol solvates of the macrolide antibiotic, 9-deoxo-11-deoxy-9,11-{imino[2-(2-methoxyethoxy)ethylidene]oxy}-(9S)-erythromycin, hereinafter referred to as dirithromycin. The stable solvates provide an efficient method of isolating pharmaceutically acceptable dirithromycin in excellent purity and yield.

Dirithromycin is a macrolide antibiotic derived from erythromycin. The antibiotic is described in Example 9 of U.S. Pat. No. 4,048,306 of Boehringer Ingelheim. The spectrum of activity of this antibiotic approximates that of erythromycin; however, dirithromycin has the advantageous property of providing high concentrations of antibiotic activity in the tissues while the plasma levels of the antibiotic remain low. To date, purification of this antibiotic has been difficult and somewhat inefficient.

J. Antibiot. 1990, 43(10), 1271-7 discloses the dissolution of dirithromycin in different solvents including acetone.

Applicants have discovered that dirithromycin exists in two forms which are distinguishable by x-ray powder diffractometry. The two forms are designated Form I and Form II. Form I dirithromycin has in essence the following x-ray powder diffraction pattern, wherein d represents the interplanar spacing and I/I₀ the relative intensity:

| d(Å) d(10⁻¹⁰m) | I/Iₒ |
|---|---|
| 11.28 | 1.00 |
| 9.81 | 0.35 |
| 8.53 | 0.76 |
| 7.67 | 0.23 |
| 7.12 | 0.02 |
| 6.94 | 0.02 |
| 6.66 | 0.10 |
| 6.39 | 0.09 |
| 5.97 | 0.21 |
| 5.65 | 0.69 |
| 5.42 | 0.67 |
| 5.18 | 0.23 |
| 4.98 | 0.08 |
| 4.83 | 0.31 |
| 4.64 | 0.07 |
| 4.43 | 0.40 |
| 4.26 | 0.17 |
| 4.14 | 0.05 |
| 4.06 | 0.15 |
| 3.86 | 0.15 |
| 3.76 | 0.17 |
| 3.62 | 0.10 |
| 3.50 | 0.08 |
| 3.43 | 0.03 |
| 3.35 | 0.07 |
| 3.04 | 0.07 |
| 2.95 | 0.02 |
| 2.88 | 0.02 |
| 2.84 | 0.02 |
| 2.71 | 0.03 |
| 2.66 | 0.02 |
| 2.58 | 0.03 |

In contrast, Form II dirithromycin has the following x-ray powder diffraction pattern, wherein d represents the interplanar spacing and I/Iₒ the relative intensity:

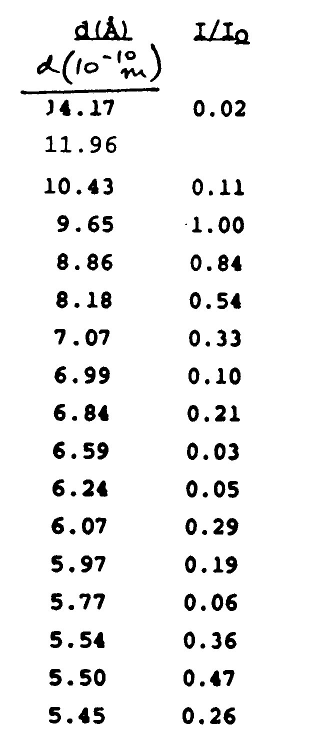

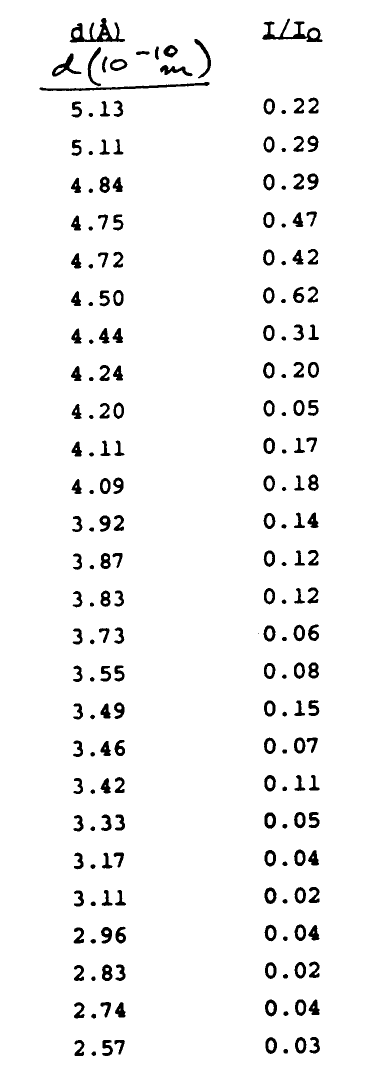

The Form I crystal can be isolated via the acetonitrile solvate of dirithromycin and exposure of the solvate to air or vacuum drying. The method of preparing Form I dirithromycin is described in U.S. Pat. No. 4,048,306 of Boehringer Ingelheim, which is hereby incorporated by reference. The disadvantage of Form I is that it is metastable. Surprisingly, the Form II crystal type is stable at ambient temperature. It is desirable to isolate the pure Form II crystal of dirithromycin to assure uniformity of product. The method of this invention provides isolated Form II dirithromycin of greatly improved quality via a convenient, efficient, and ecologically friendly isolation process.

The formation of solvates is known to be a highly individualistic effect. Dirithromycin is known to crystallize as a solvated crystal from acetonitrile; however, the acetonitrile solvate of dirithromycin is known to be unstable. See P. Lugar, R. Maier, Molecular Structure of 9-deoxy-11-deoxy-9-11-imino(2-(2-methoxyethoxy)ethylidene)oxy)-(9S)-erythromycin, 9 Journal of Crystal and Molecular Structure 329 (1979).

One aspect of this invention provides the acetone, 1-butanol, 1-propanol, and 2-propanol solvate forms of dirithromycin. The invention provides a process for isolating purified Form II dirithromycin which comprises slurrying one of the solvates of this invention in a solvent comprising from about 40% to about 100% water with stirring to produce solid, pharmaceutically pure dirithromycin. The purified Form II dirithromycin is substantially free of Form I dirithromycin.

An additional aspect of this invention further provides a process for isolating Form II dirithromycin which comprises slurrying Form I dirithromycin in a solvent comprising fro about 80% to about 100% water, wherein the solvent temperature is about 40° to about 80°C, with stirring to produce solid, pharmaceutically pure dirithromycin. Another aspect of the invention provides a process for obtaining Form II dirithromycin which comprises dissolving intermediate dirithromycin, as a solvate or Form I, in ethyl acetate, wherein the ethyl acetate temperature is from about ambient temperature to about 80°C to produce Form II dirithromycin. One additional aspect provided is purified Form II dirithromycin.

All of the aforementioned x-ray powder diffraction patterns were obtained using a Nicolet 12V powder diffractometer equipped with a graphite monochrometer with copper radiation of 1 = 0.15418 nm (1.5418/angstroms). It will be understood that the intensity values may vary due to sample preparation and instrument variations.

This invention provides an efficient process to prepare Form II dirithromycin. The Form II dirithromycin may be prepared via the acetone, 1-butanol, 1-propanol, or 2-propanol solvates of dirithromycin or from Form I. The solvates of dirithromycin, which are novel compounds, facilitate the isolation of pharmaceutically acceptable dirithromycin by removing impurities, as demonstrated by examination of the HPLC chromatogram.

The solvates may be formed by dissolving dirithro-mycin in a solvent comprised of from about 0% to 80% water and about 20% to 100% non-aqueous solvent. The non-aqueous solvent corresponds to the desired dirithromycin solvate product. Thus, when the acetonate is desired, the solvent mixture would be water and acetone. The temperature of the mixture should be from ambient temperature to about 90°C. The mixture should be stirred for about 20 minutes or more. The reaction time required will vary with the temperature of the reaction, pressure, and with the completion of reaction desired. The progress of the reaction may be followed by x-ray powder diffraction techniques. Preferred reaction conditions include a reaction temperature of about 50°C to about 92°C with a reaction time of about 30 minutes or longer. The solid solvate form of dirithromycin may be crystallized from the solution by conventional methods, including cooling or chilling, crystal seeding, evaporation of a portion of the solution, or by addition of water or organics, such as hexane, to encourage crystallization. The solid may be isolated by conventional methods including filtration and centrifugation. The isolated solid may be washed with solvent to improve purity. The solvate may be dried or used as the wet cake for subsequent reactions or isolation procedures.

Alternatively, the solvate formation may be achieved during the formation of dirithromycin. This process comprises dissolving 2-(2-methoxyethoxy)acetaldehyde or its equivalent in the form of a hydrate, or hemiacetal, in acetone, 1-butanol, 1-propanol, or 2-propanol. The solvent is chosen based on the solvate form of dirithromcyin desired. 9(S)-erythromycylamine is added to the reaction mixture with stirring. A preferred concentration of the erythromycylamine is from about 0.2 molar to about 0.7 molar. The concentration of the acetaldehyde and erythromycylamine may vary widely; however, the reaction is most efficient when the molar ratio is greater than 1.1 moles of aldehyde per 1 mole erythromycylamine. The reaction is stirred from about 30 minutes to about 20 hours. The reaction time should be based on the degree of completion desired, and may be run for a longer period of time. The reaction may be run under nitrogen atmosphere. Crystallization of the solvate may be completed by conventional methods including, but not limited to cooling, crystal seeding, and solvent evaporation. The percent recovery may be enhanced by allowing crystallization to proceed overnight and by stirring in an ice bath. The solid may be isolated by conventional methods. The isolated solid may be washed with chilled solvent to improve purity. The solid may be dried or used as the wet cake, as mentioned above.

The acetone, 1-butanol, 1-propanol, and 2-propanol solvate forms of dirithromycin may be used to isolate Form II dirithromcyin. The solvate, chosen from the four above-mentioned, is slurried in a solvent comprised of from about 80% to 100% water and from about 0% to 20% non-aqueous solvent. The non-aqueous solvent corresponds to the solvate form of the product and should be selected from acetone, 1-butanol, 1-propanol, and 2-propanol. A most preferred composition is from about 95% to about 100% water. The slurry is stirred at from about ambient temperature to about 80°C for about 2.5 hours or more. The temperature may vary based on pressure and desired speed of reaction. The completion of reaction may be monitored by x-ray powder diffractometry techniques and differential thermal analysis. The solid Form II dirithromycin may be isolated by conventional methods including vacuum filtration, simple filtration, or centrifugation. The solid may be washed with water or a solution of one of the four solvents with water and dried.

Alternatively, Form II dirithromycin may be prepared from Form I dirithromycin. Form I dirithromycin is slurried and heated, and Form II is isolated as in the process utilizing the solvate form of dirithromycin, above. The preferred temperature range is about 45°C to about 80°C. The solid product of the process is the stable Form II dirithromcyin which may be dried in a vacuum oven or by other known methods.

Alternatively, Form II may be prepared from non-solvated, dirithromycin, dirithromycin solvate or Form I dirithromycin by dissolving in ethyl acetate or toluene. The preferred concentration of dirithromycin is about 0.10 molar to about 0.28 molar. The concentration may vary with temperature, pressure, time, and degree of agitation. Crystallization of Form II dirithromycin may be completed by conventional methods including solvent evaporation, seeding, cooling, and addition of an antisolvent such as n-heptane or n-octane. The solid may be isolated by conventional methods. The isolated solid may be washed with antisolvent to improve purity.

Effective drying methods include vacuum oven drying, air oven or simple vacuum desiccator drying. The solvates are stable at ambient temperature and will tolerate vacuum drying. When vacuum oven drying is used, one must exercise caution to avoid breaking the solvate. Preferred drying conditions for the acetone solvate are about 40 to about 50°C vacuum oven drying and about 30 to about 40°C vacuum oven drying for the isopropanol solvate. Preferred drying conditions for the Form II dirithromycin are vacuum oven drying at temperatures from about 45°C to about 55°C.

It will be understood that the concentration of dirithromycin is not a critical factor in the preparation of the solvate or in the preparation of Form II dirithromcyin. A preferred concentration range for dirithromcyin in the solvate formation is from about 0.1 to about 0.2 molar. A preferred concentration range of solvate to be used in the isolation of Form II dirithromycin is from about 0.1 molar to about 0.2 molar.

The solvates of this invention are true solvates having a fixed composition of about 1 solvent molecule per molecule of dirithromycin. The solvates of this invention are particularly useful because they are stable at ambient conditions. They have been found to be very useful in the purification of dirithromcyin and as intermediates for subsequent reactions.

The following examples further illustrate the invention. The potency values reported in the following examples represent the purity of the product with respect to an anhydrous standard. Due to 5 to 10% solvent content, the reported potency values are less than the actual potency if the samples were compared to a standard with similar solvent content. The values for total related substances (TRS) were determined by HPLC analysis utilizing UV detection at 205 nm.

The following table summarizes the ¹³C NMR assignments for dirithromycin (Form I) and the four solvates. The NMR spectra of the dirithromycin solvates were recorded at 75.4 MHz at a concentration of 100 mg/ml in deuterated chloroform. The shifts are referenced to chloroform-d at 77.00 ppm. The spectra of dirithromycin were recorded at 75.4 MHz at a concentration of 50 mg/ml in deuterated chloroform with 1% tetramethylsilane. Chemical shifts are referenced to chloroform-d at 77.00 ppm. Note that a numbered structure follows the NMR spectra table.

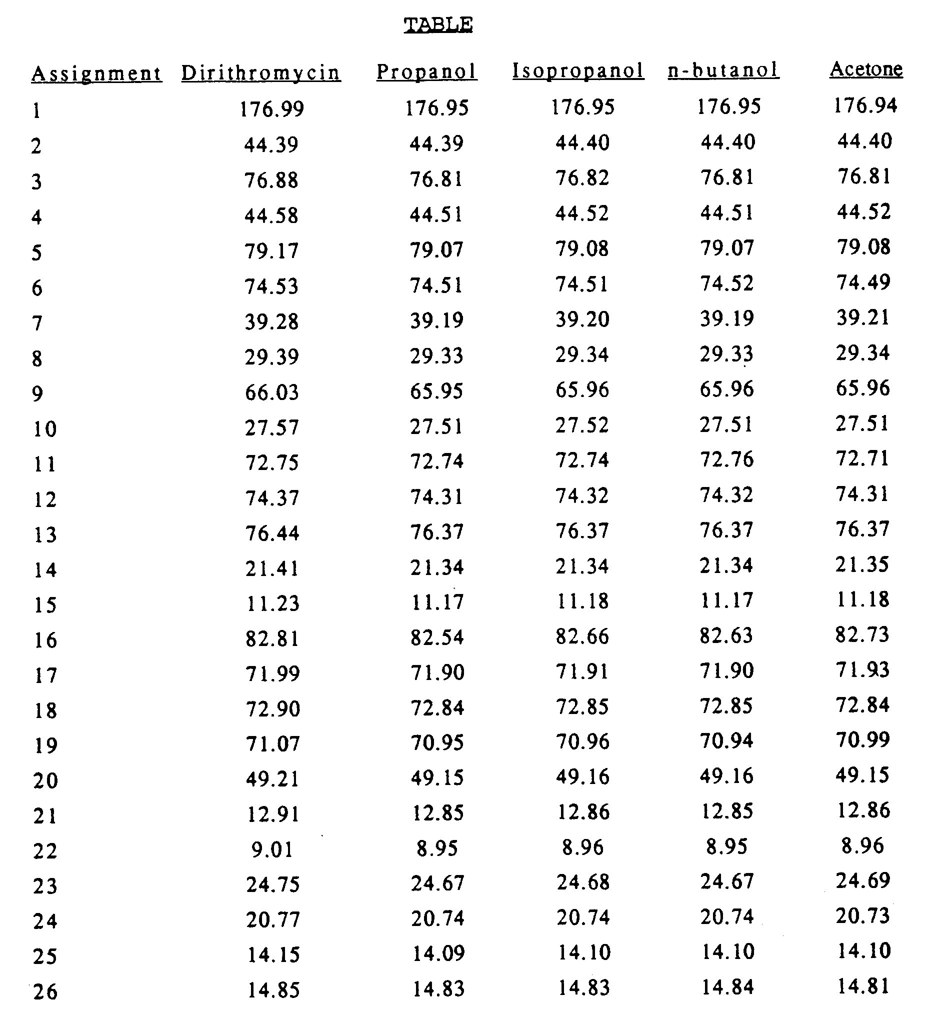

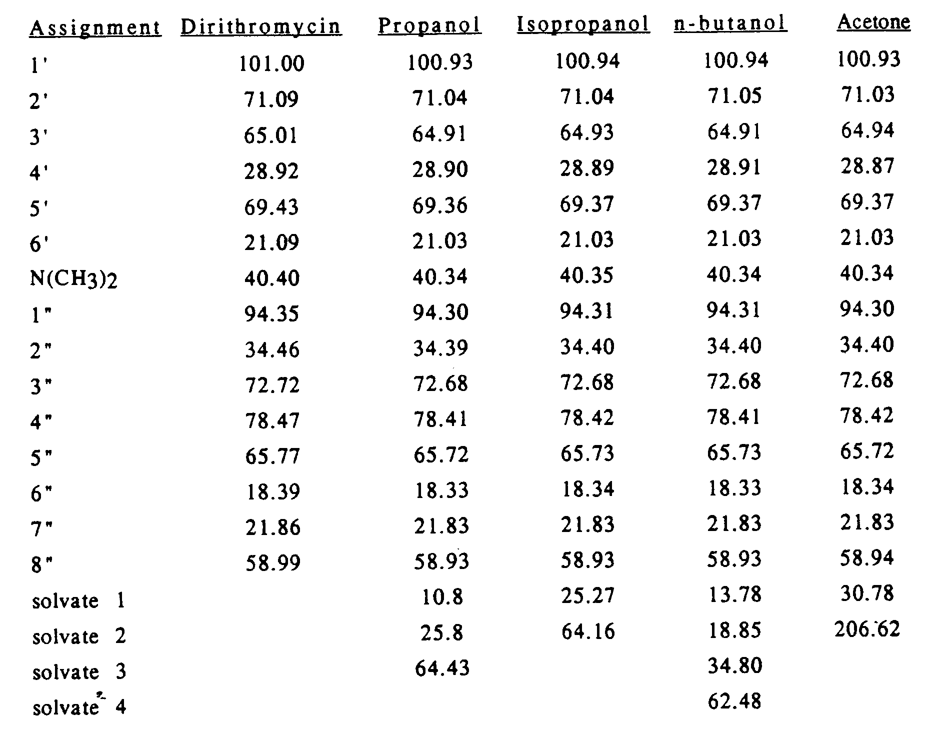

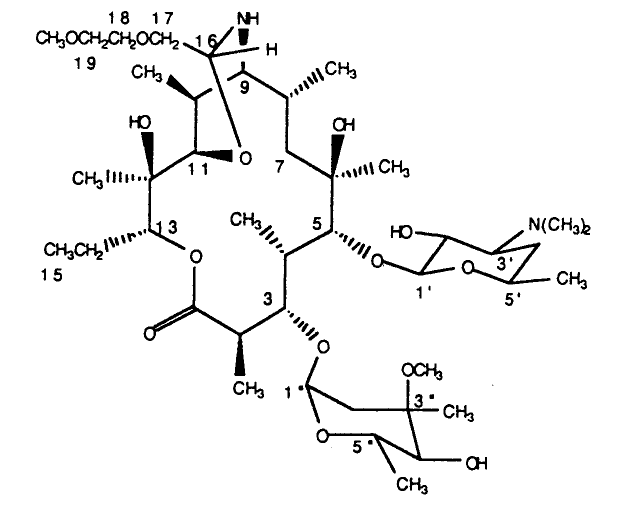

### Example 1

### Acetone Solvate of Dirithromycin

A 4.9 g. portion of 2-(2-methoxyethoxy)acetaldehyde was dissolved in 60 ml. reagent grade acetone. A 20.0 g. portion of (9S)-erythromycylamine A was added to the reaction mixture with stirring. The mixture was stirred for 2 hours in a nitrogen atmosphere at room temperature. The mixture was seeded with dirithromycin crystals and stirred overnight at room temperature.

The reaction mixture was stirred for 1 hour in an ice bath. The solid was isolated by filtration and was washed with 20.0 ml. of cold acetone. The wet cake of solid was resuspended in 40 ml. of acetone. The reslurried mixture was stirred at about 0°C to about 5°C for 1 hour. The solid was isolated by filtration and washed with 20.0 ml. of cold acetone. The solid was dried in a vacuum oven at 50°C overnight. The product was identified as the acetone solvate of dirithromycin by x-ray powder diffractometry and NMR spectroscopy. The total yield of the compound was 17.68 g. (77.8%)
Potency: 91.6%
TRS: 2.9% (reduced from 12.2% before resuspension).

### Example 2

### Acetone Solvate of Dirithromycin

A 10.0 g. sample of dirithromycin was added to a 30 ml. solvent mixture comprised of 90% acetone and 10% water. The reaction mixture was heated to reflux and 10 additional ml. of solvent were added to dissolve remaining solids. The clear, colorless solution was stirred while adding 68 ml. of 58-60°C water. The reaction mixture was stirred 0.5 hour at about 58°C. The mixture was allowed to cool to room temperature (about 2 hours). The cooled mixture was placed in an ice bath and stirred for 2 hours. The solid was isolated by filtration, and rinsed with a 20 ml. chilled mixture of 67% water: 33% acetone. The isolated solid was dried in vacuo at 40°C overnight. The product was identified as the acetone solvate of dirithromycin by x-ray powder diffractometry.
Total yield: 87.1%
Potency: 87.3%
TRS: 1.92% (reduced from 4.51% in the starting dirithromycin)

### Example 3

### Acetone Solvate of Dirithromycin

A 10.0 g. sample of dirithromycin was added to a 55 ml. solvent mixture comprised of 90% acetone and 10% water. The reaction mixture was heated and 23 ml of solvent was distilled away. The clear, colorless solution was stirred at 58-60°C for 0.5 hour. A 52 ml portion of water was added in small aliquots over the 30 minutes of heated stirring. The reaction mixture was allowed to cool to room temperature. The cooled mixture was placed in an ice bath and stirred for 0.75 hours. The solid was isolated by filtration, and rinsed with a 20 ml. chilled mixture of 67% water: 33% acetone. The isolated solid was dried in vacuo at 40°C over night. The product was identified as the acetone solvate of dirithromycin by x-ray powder diffractometry.
Total yield: 92.6%
Potency: 89.0%
TRS: 1.93% (reduced from 4.51% in starting dirithromycin)

### Example 4

### Acetone Solvate of Dirithromycin

A 9 g. portion of Form I dirithromycin was added to 27 ml. acetone. The reaction mixture was heated to 50°C and stirred for 2.5 hours. The reaction mixture was allowed to cool to 5°C and the mixture was stirred for 40 minutes at 5°C. The solid was isolated by filtration and washed with 20 ml. of 5°C acetone. The sample was dried in a vacuum oven at 50°C. The product was identified as the acetone solvate of dirithromycin by x-ray powder diffractometry.
Yield: 77%

### Example 5

### 1-Propanol Solvate of Dirithromycin

A 5 g. portion of dirithromycin was added to a solvent mixture comprised of 5 ml. water and 23 ml. 1-propanol. The solution was heated to boiling and allowed to boil until the reaction volume was 10 ml. Dirithromycin seed crystals were added to the mixture with stirring. The reaction mixture was allowed to cool to room temperature and 20 ml. of water were added to the sample with stirring. The solid was isolated by filtration and washed with 3 washes, each of 50 ml. water. The sample was dried in a vacuum dessicator. The product was identified as the 1-propanol solvate by x-ray powder diffractometry and NMR spectroscopy.

### Example 6

### 1-Propanol Solvate of Dirithromycin

A 10.0 g. sample of dirithromycin was dissolved in a solvent mixture comprised of 18 ml. of 1-propanol and 12 ml. of water. The solvent was heated to about 50°C to dissolve remaining solids. The solution was stirred at about 50°C for 0.5 hour. An additional 30 ml. portion of water was added slowly over the 30 minutes of heated stirring. The reaction mixture was allowed to cool to room temperature. The cooled mixture was placed in an ice bath and stirred for 1.0 hour. The solid was isolated by filtration, and rinsed with a 20 ml. chilled mixture of 67% water and 33% 1-propanol. The isolated solid was dried in vacuo at 35°C over night. The solid was identified as the propanol solvate of dirithromycin by ¹H NMR spectroscopy.
Total yield: 77.6%
Potency: 89.9%

### Example 7

### 2-Propanol Solvate of Dirithromycin

A 5 g. portion of dirithromycin was added to a solvent mixture comprised of 5 ml. water and 23 ml. 2**-**propanol. The solution was heated to 55°C to allow the solid to dissolve. The reaction mixture was heated to 83°C and allowed to boil until the reaction volume was 10 ml. Dirithromycin seed crystals were added to the mixture with stirring. The reaction mixture was allowed to cool to room temperature and 3 ml. of water were added to the sample with stirring. The solid was isolated by filtration and washed with 3 washes, each of 50 ml of water. The product was dried and was identified as the 2-propanol solvate by x-ray powder diffractometry and NMR spectroscopy.

### Example 8

### 1-Butanol Solvate of Dirithromycin

A 5 g. portion of dirithromycin was added to a solvent mixture comprised of 5 ml. water and 40 ml. 1-butanol. The solution was heated to boiling and allowed to boil until the reaction volume was 15 ml. The reaction mixture was cooled to room temperature with stirring. Dirithromycin seed crystals were added to the stirring mixture. The reaction mixture was stirred for 10 minutes and 40 ml. of hexane was added to the reaction mixture to precipitate the solvate. The mixture was stirred for 5 minutes at room temperature. The solid was isolated by filtration and washed with 3 washes, each of 50 ml. of deionized water. The sample was dried in a vacuum desiccator. The product was identified as the 1-butanol solvate by x-ray powder diffraction and NMR spectroscopy.

### Example 9

### Isolation of Dirithromycin Form II From the Acetonate

A 20 g. portion of non-solvated dirithromycin was added to a solvent mixture comprised of 81 ml. acetone and 9 ml. water. The solution was heated to about 60°C. The solution was maintained at 60°C until the reaction volume was 35 ml. An additional 100 ml. portion of water was added slowly over 1 hour. The mixture was allowed to cool to room temperature. The cooled mixture was placed in an ice bath and stirred for 1 hour. The solid was isolated by filtration, and rinsed with 25 ml. of chilled solvent comprised of 67% water and 33% acetone. The solid was rinsed with a 40 ml. portion of water at ambient temperature. The wet cake of acetone solvate of dirithromycin was left at ambient conditions overnight.

A 180 ml. portion of water was added to the wet cake of acetone solvate. The mixture was heated to 70°C, and was stirred at 70°C with a nitrogen purge for 4 hours. The solid was immediately isolated by filtration and rinsed with a 30 ml. portion of water which had been warmed to 70°C. The isolated solid was dried in vacuo at 40°C overnight. The solid was identified as Form II dirithromycin by x-ray powder diffractometry.
Total yield: 90.8%
Potency: 96.05%
TRS: 3.04% (Reduced from 4.53% in the non-solvated dirithromycin).

### Example 10

### Isolation of Form II Dirithromycin

A 10.0 g. sample of acetone solvate of dirithromycin was added with stirring to 100 ml. of water with nitrogen purge. The temperature of the reaction mixture was increased to 74°C and stirred at about 72-75°C for 4 hours. The warm mixture was vacuum filtered and washed with about 35 ml. of 60°C water. The solid was dried in vacuo at 50°C overnight. The solid was identified as Form II dirithromycin, by x-ray powder diffractometry. The total yield of the reaction was 8.74 g. (87.4%).
Potency: 90.7%
Acetone: < 0.03%
The process of Example 8 was repeated with the following results:
Total yield: 8.77 g. (87.7%)
Potency: 93.2%
Acetone: < 0.03%

### Example 11

### Isolation of Form II Dirithromycin

A 15 g. portion of Form I dirithromycin was added to 150 ml. water. The reaction mixture was heated to 74°C and stirred for 4 hours at 74°C. The solid was isolated by filtration and washed with two washes, each of 40 ml. of 70°C water. The sample was dried in a vacuum oven at 25°C for about 68 hours. The product was identified as Form II dirithromycin by x-ray powder diffractometry.
Yield: 97.5%
Potency: 96.0%
TRS: 3.8%

### Example 12

### Isolation of Form II Dirithromycin

A 5 g. portion of Form I dirithromycin was added to 25 ml. ethyl acetate. The solution was heated to 76°C and allowed to boil until the reaction volume was about 15 ml. The reaction mixture was allowed to cool to room temperature and 20 ml. of water were added to the sample with stirring. The solid was isolated by filtration and washed with three washes, each of 25 ml. water. The sample was washed with one wash of heptane. The product was identified as Form II dirithromycin by x-ray powder diffractometry.

### Example 13

### Isolation of Form II Dirithromycin

A 3.01 g. portion of Form I dirithromycin was added to 15 ml. ethyl acetate. The reaction mixture was heated to about 76°C and the mixture was allowed to boil until the reaction volume was about 10 ml. A 20 ml. portion of n-octane was added. The mixture was allowed to cool to room temperature. The solid was isolated by filtration. The sample was dried at room temperature. The product was identified as Form II dirithromycin by x-ray powder diffractometry.
Total Yield: 95%

## Claims (Claims for the following Contracting State(s): AT, BE, CH, DE, DK, FR, GB, IT, LI, LU, NL, PT, SE)

1. A crystalline acetone, 1-butanol, 1-propanol or 2-propanol solvate of dirithromycin.

2. An acetone solvate of dirithromycin as claimed in Claim 1.

3. A 1-butanol solvate of dirithromycin as claimed in Claim 1.

4. Purified crystalline Form II dirithromycin having the following x-ray powder diffraction pattern:
| d(Å) d(10⁻¹⁰m) | d(Å) d(10⁻¹⁰m) |
|---|---|
| 14.17 | 4.72 |
| 11.96 | 4.50 |
| 10.43 | 4.44 |
| 9.65 | 4.24 |
| 8.86 | 4.20 |
| 8.18 | 4.11 |
| 7.07 | 4.09 |
| 6.99 | 3.92 |
| 6.84 | 3.87 |
| 6.59 | 3.83 |
| 6.24 | 3.73 |
| 6.07 | 3.55 |
| | 3.49 |
| 5.97 | 3.46 |
| 5.77 | 3.42 |
| 5.54 | 3.33 |
| 5.50 | 3.17 |
| 5.45 | 3.11 |
| 5.13 | 2.96 |
| 5.11 | 2.83 |
| 4.84 | 2.74 |
| 4.75 | 2.57 |

5. A process for preparing Form II dirithromycin as claimed in Claim 4, which comprises slurrying a solvate as claimed in any one of Claims 1 to 4, in a solvent comprising from 40% to 100% water.

6. A process for isolating Form II dirithromycin, as claimed in Claim 4, which comprises slurrying Form I dirithromycin in a solvent comprising from 80% to 100% water, wherein the solvent temperature is from 40°C to 80°C.

7. A process for obtaining Form II dirithromycin, as claimed in Claim 4, which comprises dissolving intermediate dirithromycin in ethyl acetate, wherein the ethyl acetate temperature is from ambient temperature to 80°C.

8. A process for preparing a solvate as claimed in any one of Claims 1 to 3, which comprises contacting dirithromycin with a solvent comprised of from 0% to 80% water and from 20% to 100% nonaqueous solvent; wherein said nonaqueous solvent corresponds to the desired solvate; or reacting an appropriate aldehyde dissolved in a nonaqueous solvent; wherein said nonaqueous solvent corresponds to the desired solvate; with an appropriate amine; followed by crystallization of the solvate.

## Claims (Claims for the following Contracting State(s): ES)

1. A process for preparing Form II dirithromycin which process comprises slurrying an acetone solvate in a solvent comprising from 40% to 100% water.

2. A process for preparing Form II dirithromycin which process comprises slurrying a propanol or 1-butanol solvate in a solvent comprising from 40% to 100% water.

3. A process for isolating Form II dirithromycin which process comprises slurrying Form I dirithromycin in a solvent comprising from 80% to 100% water, wherein the solvent temperature is from 40°C to 80°C.

4. A process for obtaining Form II dirithromycin which process comprises dissolving intermediate dirithromycin in ethyl acetate, wherein the ethyl acetate temperature is from ambient temperature to 80°C.

5. A process for preparing an acetone, 1-butanol, 1-propanol or 2-propanol solvate which comprises contacting dirithromycin with a solvent comprised of from 0% to 80% water and from 20% to 100% nonaqueous solvent; wherein said nonaqueous solvent corresponds to the desired solvate; or reacting an appropriate aldehyde dissolved in a nonaqueous solvent; wherein said nonaqueous solvent corresponds to the desired solvate; with an appropriate amine; followed by crystallization of the solvate.

## Claims (Claims for the following Contracting State(s): GR)

1. A process for preparing Form II dirithromycin which process comprises slurrying an acetone solvate in a solvent comprising from 40% to 100% water.

2. A process for preparing Form II dirithromycin which process comprises slurrying a propanol or 1-butanol solvate in a solvent comprising from 40% to 100% water.

3. A process for isolating Form II dirithromycin which process comprises slurrying Form I dirithromycin in a solvent comprising from 80% to 100% water, wherein the solvent temperature is from 40°C to 80°C.

4. A process for obtaining Form II dirithromycin which process comprises dissolving intermediate dirithromycin in ethyl acetate, wherein the ethyl acetate temperature is from ambient temperature to 80°C.

5. A process for preparing an acetone, 1-butanol, 1-propanol or 2-propanol solvate which comprises contacting dirithromycin with a solvent comprised of from 0% to 80% water and from 20% to 100% nonaqueous solvent; wherein said nonaqueous solvent corresponds to the desired solvate; or reacting an appropriate aldehyde dissolved in a nonaqueous solvent; wherein said nonaqueous solvent corresponds to the desired solvate; with an appropriate amine; followed by crystallization of the solvate.

6. A crystalline acetone, 1-butanol, 1-propanol or 2-propanol solvate of dirithromycin.

7. An acetone solvate of dirithromycin as claimed in Claim 6.

8. A 1-butanol solvate of dirithromycin as claimed in Claim 6.

## Patentansprüche (Patentansprüche für folgende(n) Vertragsstaat(en): AT, BE, CH, DE, DK, FR, GB, IT, LI, LU, NL, PT, SE)

1. Kristallines Aceton-, 1-Butanol-, 1-Propanol- oder 2-Propanolsolvat von Dirithromycin.

2. Acetonsolvat von Dirithromycin gemäß Anspruch 1.

3. 1-Butanolsolvat von Dirithromycin gemäß Anspruch 1.

4. Gereinigtes kristallines Form II Dirithromycin, das das folgende Röntgenbeugungsmuster in Pulverform aufweist:
| d(Å) d(10⁻¹⁰m) | d(Å) d(10⁻¹⁰m) | d(Å) d(10⁻¹⁰m) | d(Å) d(10⁻¹⁰m) |
|---|---|---|---|
| 14,17 | 6,07 | 4,50 | 3,49 |
| 11,96 | 5,97 | 4,44 | 3,46 |
| 10,43 | 5,77 | 4,24 | 3,42 |
| 9,65 | 5,54 | 4,20 | 3,33 |
| 8,86 | 5,50 | 4,11 | 3,17 |
| 8,18 | 5,45 | 4,09 | 3,11 |
| 7,07 | 5,13 | 3,92 | 2,96 |
| 6,99 | 5,11 | 3,87 | 2,83 |
| 6,84 | 4,84 | 3,83 | 2,74 |
| 6,59 | 4,75 | 3,73 | 2,57 |
| 6,24 | 4,72 | 3,55 | |

5. Verfahren zur Herstellung von Form II Dirithromycin gemäß Anspruch 4, dadurch gekennzeichnet, daß ein Solvat nach einem der Ansprüche 1 bis 4 in einem Lösemittel, das 40% bis 100% Wasser enthält, aufgeschlämmt wird.

6. Verfahren zur Isolierung von Form II Dirithromycin gemäß Anspruch 4, dadurch gekennzeichnet, daß Form I Dirithromycin in einem Lösemittel, das 80% bis 100% Wasser enthält, und eine Temperatur von 40°C bis 80°C aufweist, aufgeschlämmt wird.

7. Verfahren zur Herstellung von Form II Dirithromycin gemäß Anspruch 4, dadurch gekennzeichnet, daß ein Dirithromycinzwischenprodukt in Ethylacetat, das eine Temperatur von Umgebungstemperatur bis 80°C aufweist, gelöst wird.

8. Verfahren zur Herstellung eines Solvats gemäß einem der Ansprüche 1 bis 3, dadurch gekennzeichnet, daß Dirithromycin mit einem Lösemittel, das 0% bis 80% Wasser und 20% bis 100% eines nicht wäßrigen Lösemittels enthält, wobei das nicht wäßrige Lösemittel dem gewünschten Solvat entspricht, behandelt wird oder ein in einem nicht wäßrigen Lösemittel gelöster geeigneter Aldehyd, wobei das nicht wäßrige Lösemittel dem gewünschten Solvat entspricht, mit einem geeigneten Amin umgesetzt und das gewünschte Solvat dann auskristallisiert wird.

## Patentansprüche (Patentansprüche für folgende(n) Vertragsstaat(en): ES)

1. Verfahren zur Herstellung von Form II Dirithromycin, dadurch gekennzeichnet, daß ein Acetonsolvat in einem Lösemittel, das 40% bis 100% Wasser enthält, aufgeschlämmt wird.

2. Verfahren zur Herstellung von Form II Dirithromycin, dadurch gekennzeichnet, daß ein Propanol- oder 1-Butanolsolvat in einem Lösemittel, das 40% bis 100% Wasser enthält, aufgeschlämmt wird.

3. Verfahren zur Isolierung von Form II Dirithromycin, dadurch gekennzeichnet, daß Form I Dirithromycin in einem Lösemittel, das 80% bis 100% Wasser enthält und eine Temperatur von 40°C bis 80°C aufweist, aufgeschlämmt wird.

4. Verfahren zur Herstellung von Form II Dirithromycin, dadurch gekennzeichnet, daß ein Dirithromycinzwischenprodukt in Ethylacetat, das eine Temperatur von Umgebungstemperatur bis 80°C aufweist, gelöst wird.

5. Verfahren zur Herstellung eines Aceton-, 1-Butanol-, 1-Propanol oder 2-Propanolsolvats, dadurch gekennzeichnet, daß Dirithromycin mit einem Lösemittel, das 0% bis 80% Wasser und 20% bis 100% eines nicht wäßrigen Lösemittels enthält, wobei das nicht wäßrige Lösemittel dem gewünschten Solvat entspricht, behandelt wird oder ein in einem nicht wäßrigen Lösemittel gelöster geeigneter Aldehyd, wobei das nicht wäßrige Lösemittel dem gewünschten Solvat entspricht, mit einem geeigneten Amin umgesetzt und das gewünschte Solvat dann auskristallisiert wird.

## Patentansprüche (Patentansprüche für folgende(n) Vertragsstaat(en): GR)

1. Verfahren zur Herstellung von Form II Dirithromycin, dadurch gekennzeichnet, daß ein Acetonsolvat in einem Lösemittel, das 40% bis 100% Wasser enthält, aufgeschlämmt wird.

2. Verfahren zur Herstellung von Form II Dirithromycin, dadurch gekennzeichnet, daß ein Propanol- oder 1-Butanolsolvat in einem Lösemittel, das 40% bis 100% Wasser enthält, aufgeschlämmt wird.

3. Verfahren zur Isolierung von Form II Dirithromycin, dadurch gekennzeichnet, daß Form I Dirithromycin in einem Lösemittel, das 80% bis 100% Wasser enthält und eine Temperatur von 40°C bin 80°C aufweist, aufgeschlämmt wird.

4. Verfahren zur Herstellung von Form II Dirithromycin, dadurch gekennzeichnet, daß ein Dirithromycinzwischenprodukt in Ethylacetat, das eine Temperatur von Umgebungstemperatur bis 80°C aufweist, gelöst wird.

5. Verfahren zur Herstellung eines Aceton-, 1-Butanol-, 1-Propanol oder 2-Propanolsolvats, dadurch gekennzeichnet, daß Dirithromycin mit einem Lösemittel, das 0% bis 80% Wasser und 20% bis 100% eines nicht wäßrigen Lösemittels enthält, wobei das nicht wäßrige Lösemittel dem gewünschten Solvat entspricht, behandelt wird oder ein in einem nicht wäßrigen Lösemittel gelöster geeigneter Aldehyd, wobei das nicht wäßrige Lösemittel dem gewünschten Solvat entspricht, mit einem geeigneten Amin umgesetzt und das gewünschte Solvat dann auskristallisiert wird.

6. Kristallines Aceton-, 1-Butanol-, 1-Propanol- oder 2-Propanolsolvat von Dirithromycin.

7. Acetonsolvat von Dirithromycin gemäß Anspruch 6.

8. 1-Butanolsolvat von Dirithromycin gemäß Anspruch 6.

## Revendications (Revendications pour l'(les) Etat(s) contractant(s) suivant(s): AT, BE, CH, DE, DK, FR, GB, IT, LI, LU, NL, PT, SE)

1. Solvate d'acétone, de 1-butanol, de 1-propanol ou de 2-propanol cristallin de la dirithromycine.

2. Solvate d'acétone de la dirithromycine selon la revendication 1.

3. Solvate de 1-butanol de la dirithromycine selon la revendication 1.

4. Forme cristalline II purifiée de la dirithromycine présentant le diagramme de diffraction de la poudre par rayons X suivant :
| d (Å) d(10⁻¹⁰m) | d (Å) d(10⁻¹⁰m) |
|---|---|
| 14,17 | 4,72 |
| 11,96 | 4,50 |
| 10,43 | 4,44 |
| 9,65 | 4,24 |
| 8,86 | 4,20 |
| 8,18 | 4,11 |
| 7,07 | 4,09 |
| 6,99 | 3,92 |
| 6,84 | 3,87 |
| 6,59 | 3,83 |
| 6,24 | 3,73 |
| 6,07 | 3,55 |
| 5,97 | 3,49 |
| 5,77 | 3,46 |
| 5,54 | 3,42 |
| 5,50 | 3,33 |
| 5,45 | 3,17 |
| 5,13 | 3,11 |
| 5,11 | 2,96 |
| 4,84 | 2,83 |
| 4,75 | 2,74 |
| | 2,57 |

5. Procédé pour la préparation de la Forme II de la dirithromycine selon la revendication 4, qui comprend la mise en suspension d'un solvate selon l'une quelconque des revendications 1 à 4, dans un solvant comprenant de 40 % à 100 % d'eau.

6. Procédé pour l'isolement de la Forme II de la dirithromycine, selon la revendication 4, qui comprend la mise en suspension de la Forme I de la dirithromycine dans un solvant comprenant de 80 % à 100 % d'eau, dans lequel la température du solvant est de 40 °C à 80 °C.

7. Procédé pour l'obtention de la Forme II de la dirithromycine, selon la revendication 4, qui comprend la dissolution de l'intermédiaire de dirithromycine dans l'acétate d'éthyle, dans lequel la température de l'acétate d'éthyle se situe entre la température ambiante et 80 °C.

8. Procédé pour la préparation d'un solvate selon l'une quelconque des revendications 1 à 3, qui comprend la mise en contact de la dirithromycine avec un solvant comprenant de 0 % à 80 % d'eau et de 20 % à 100 % d'un solvant non-aqueux; dans lequel ledit solvant non-aqueux correspond au solvate souhaité; ou la mise en réaction d'un aldéhyde approprié dissous dans un solvant non-aqueux, dans lequel ledit solvant non-aqueux correspond au solvate souhaité; avec une amine appropriée; suivie par la cristallisation du solvate.

## Revendications (Revendications pour l'(les) Etat(s) contractant(s) suivant(s): ES)

1. Procédé pour la préparation de la Forme II de la dirithromycine, procédé qui comprend la mise en suspension d'un solvate d'acétone dans un solvant comprenant de 40 % à 100 % d'eau.

2. Procédé pour la préparation de la Forme II de la dirithromycine, procédé qui comprend la mise en suspension d'un solvate de propanol ou de 1-butanol dans un solvant comprenant de 40 % à 100 % d'eau.

3. Procédé pour l'isolement de la Forme II de la dirithromycine, procédé qui comprend la mise en suspension de la Forme I de la dirithromycine dans un solvant comprenant de 80 % à 100 % d'eau, dans lequel la température du solvant est de 40 °C à 80 °C.

4. Procédé pour l'obtention de la Forme II de la dirithromycine, procédé qui comprend la dissolution de l'intermédiaire de dirithromycine dans l'acétate d'éthyle, dans lequel la température de l'acétate d'éthyle se situe entre la température ambiante et 80 °C.

5. Procédé pour la préparation d'un solvate d'acétone, de 1-butanol, de 1-propanol ou de 2-propanol, qui comprend la mise en contact de la dirithromycine avec un solvant comprenant de 0 % à 80 % d'eau et de 20 % à 100 % d'un solvant non-aqueux; dans lequel ledit solvant non-aqueux correspond au solvate souhaité; ou la mise en réaction d'un aldéhyde approprié dissous dans un solvant non-aqueux; dans lequel ledit solvant non-aqueux correspond au solvate souhaité; avec une amine appropriée; suivie par la cristallisation du solvate.

## Revendications (Revendications pour l'(les) Etat(s) contractant(s) suivant(s): GR)

1. Procédé pour la préparation de la Forme II de la dirithromycine, procédé qui comprend la mise en suspension d'un solvate d'acétone dans un solvant comprenant de 40 % à 100 % d'eau.

2. Procédé pour la préparation de la Forme II de la dirithromycine, procédé qui comprend la mise en suspension d'un solvate de propanol ou de 1-butanol dans un solvant comprenant de 40 % à 100 % d'eau.

3. Procédé pour l'isolement de la Forme II de la dirithromycine, procédé qui comprend la mise en suspension de la Forme I de la dirithromycine dans un solvant comprenant de 80 % à 100 % d'eau, dans lequel la température du solvant est de 40 °C à 80 °C.

4. Procédé pour l'obtention de la Forme II de la dirithromycine, procédé qui comprend la dissolution de l'intermédiaire de dirithromycine dans l'acétate d'éthyle, dans lequel la température de l'acétate d'éthyle se situe entre la température ambiante et 80 °C.

5. Procédé pour la préparation d'un solvate d'acétone, de 1-butanol, de 1-propanol ou de 2-propanol, qui comprend la mise en contact de la dirithromycine avec un solvant comprenant de 0 % à 80 % d'eau et de 20 % à 100 % d'un solvant non-aqueux; dans lequel ledit solvant non-aqueux correspond au solvate souhaité; ou la mise en réaction d'un aldéhyde approprié dissous dans un solvant non-aqueux; dans lequel ledit solvant non-aqueux correspond au solvate souhaité; avec une amine appropriée; suivie par la cristallisation du solvate.

6. Solvate d'acétone, de 1-butanol, de 1-propanol ou de 2-propanol cristallin de la dirithromycine.

7. Solvate d'acétone de la dirithromycine selon la revendication 6.

8. Solvate de 1-butanol de la dirithromycine selon la revendication 6.
